# EUROPÄISCHE PATENTSCHRIFT

(11) **EP 0 992 246 B1**
(45) Veröffentlichungstag und Bekanntmachung des Hinweises auf die Patenterteilung: **08.12.2004**
(21) Anmeldenummer: 99119407.7
(22) Anmeldetag: 30.09.1999
(51) Int. Cl.: A61L 2/04, B65B 55/06

(54) **Sterilisiertunnel**
Sterilisation tunnel
Tunnel de stérilisation

(30) Priorität: 08.10.1998 DE 19846277
(43) Veröffentlichungstag der Anmeldung: 12.04.2000
(73) Patentinhaber: ROBERT BOSCH GMBH, 70442 Stuttgart (DE)
(72) Erfinder: Pennekamp, Ingbert, 74564 Crailsheim (DE); Windsheimer, Manfred, 74589 Satteldorf (DE)

(56) Entgegenhaltungen:
- EP-A- 0 570 946
- DE-A- 2 214 080
- DE-A- 2 735 532
- DE-A- 4 131 258

## Beschreibung

### Stand der Technik

Die Erfindung betrifft einen Sterilisiertunnel nach dem Oberbegriff des Anspruchs 1. Ein derartiger Sterilisiertunnel muß nach dem Durchlaufen einer Behältercharge oder am Ende einer Produktionsschicht leergefahren werden, um bei der Wiederaufnahme der Produktion sicherzustellen, daß sich keine Verpackungsbehälter einer vorhergehenden Charge mehr im Sterilisiertunnel befinden, die zwischenzeitlich kontaminiert sein könnten. Dazu wird in der Regel am Behälterstromende in der Einlaufzone des Sterilisiertunnels ein balkenförmiger Leerfahrschieber auf die als Förderband ausgebildete Transporteinrichtung für die Behälter gelegt, der die vor ihm befindlichen Behälter oder Behälterteile durch den Sterilisiertunnel durchschiebt. Kritisch ist jedoch der Auslaufbereich innerhalb des Sterilisiertunnels vor dessen Ausgangsschleuse, bei dem die Behälter von dem Umlenkbereich der Transporteinrichtung auf ein Ausführblech überführt werden. An dieser Stelle stoppt der Leerfahrschieber und die vor ihm angeordneten Behälter, weil er von keinem nachfolgenden Element weitergeschoben wird. Bekannt sind daher aufwendige mechanische Lösungen wie Ausschieberechen o.ä., die von außen in den Auslaufbereich des Sterilisiertunnels eingebracht werden müssen, um den Leerfahrschieber und die restlichen Behälter vollens auszuschleusen. Um bei sterilisierbaren Auslaufbereichen eine Kontamination auszuschließen, ist es daher je nach der Art der konstruktiven Lösung oftmals erforderlich, diese Teile vor dem Einbringen in den Auslaufbereich zu sterilisieren.

### Vorteile der Erfindung

Der erfindungsgemäße Sterilisiertunnel mit den kennzeichnenden Merkmalen des Anspruchs 1 hat demgegenüber den Vorteil, daß das Leerfahren des Sterilisiertunnels auf besonders einfache Weise manuell oder automatisch erfolgen kann. Da von außerhalb keine Teile in die Steril- bzw. Kühlzone des Sterilisiertunnels eingeführt werden müssen, ergibt sich eine besonders einfache Handhabung, da keine Teile zusätzlich vorsterilisiert werden müssen.

Weitere Vorteile und vorteilhafte Weiterbildungen des erfindungsgemäßen Sterilisiertunnels ergeben sich aus den Unteransprüchen und der Beschreibung.

### Zeichnung

Ein Ausführungsbeispiel der Erfindung ist in der Zeichnung dargestellt und wird nachfolgend näher erläutert. Es zeigen:
Figur 1 einen vereinfachten Längsschnitt durch einen Sterilisiertunnel und die
Figuren 2 bis 4 die Kühlzone des Sterilisiertunnels gemäß Figur 1 während verschiedenen Arbeitsphasen der Leerfahreinrichtung.

### Beschreibung des Ausführungsbeispiels

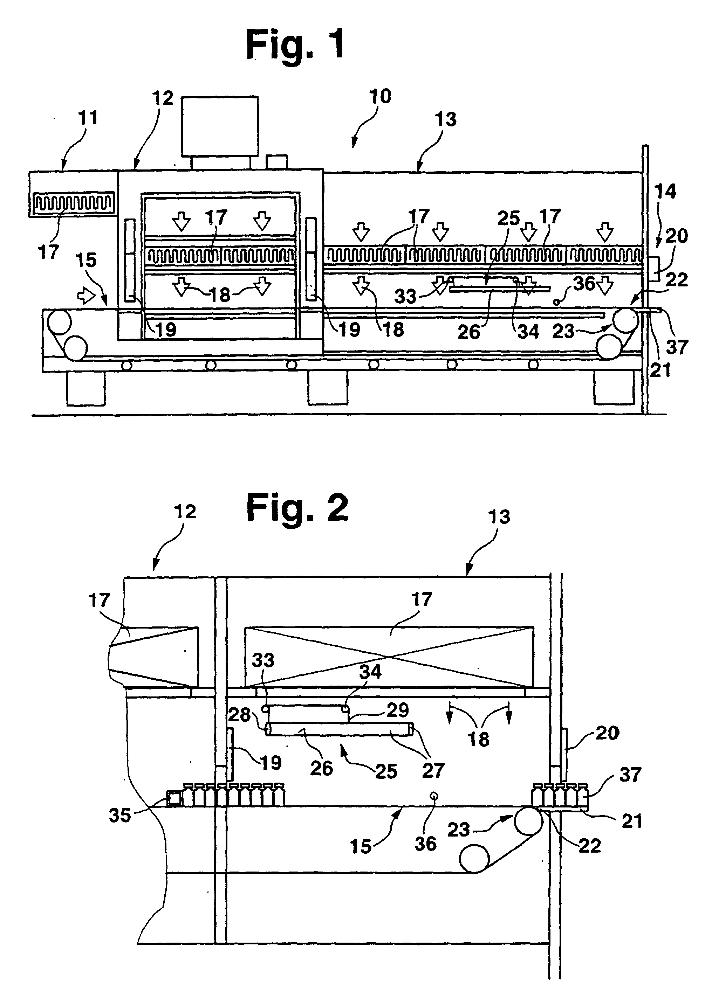

Der in der Figur 1 schematisch dargestellte Sterilisiertunnel 10 besteht im wesentlichen aus einer Einlaufzone 11, einer Sterilisierzone 12 und einer Kühlzone 13. Pharmazeutische Behältnisse wie Ampullen, Vials 1 o.ä., die von einer Reinigungsmaschine kommen, werden in der Einlaufzone 11 dem Sterilisiertunnel 10 zugeführt und verlassen diesen an seinem entgegengesetzten Ende 14 nach der Kühlzone 13, um in nachfolgenden, nicht dargestellten Füll- und Verschließmaschinen weiterverarbeitet zu werden.

Zum Transport der Vials 1 durch die einzelnen Zonen innerhalb des Sterilisiertunnels 10 dient ein horizontal umlaufendes, endloses Transportband 15. In der Praxis sind derartige Transportbänder als luftdurchlässige Drahtgeflechtbänder aus Edelstahl ausgebildet. Oberhalb des Transportbandes 15 sind in den verschiedenen Zonen des Sterilisiertunnels 10 großflächige Filterelemente 17 angeordnet, die von nicht dargestellten Ventilatoren erzeugte und von Heizeinrichtungen erwärmte Luftströme 18 derart ausrichten, daß die Luftströmungen 18 als sogenannte Laminar Flow-Strömungen senkrecht zur Transportrichtung der Vials 1 diese umströmen und anschließend umgewälzt werden. Da die Luftströme 18 in den einzelnen Zonen unterschiedliche Temperaturen aufweisen, wobei die Temperatur in der Sterilzone 12 am höchsten ist, sind zwischen den Zonen höhenverstellbare Zwischenwände 19 angeordnet, um ein Überströmen der unterschiedlich temperierten Luft zwischen den einzelnen Zonen zu reduzieren oder zu verhindern.

Im Bereich des Endes 14 der Kühlzone 13 ist unterhalb einer ebenfalls höhenverstellbaren Austrittsschleuse 20 ein Überführblech 21 angeordnet. Das Überführblech 21 ragt mit seinem einen Ende 22 bis unmittelbar an den Umlenkbereich 23 des Transportbandes 15 innerhalb des Sterilisiertunnels 10 heran. An das aus dem Sterilisiertunnel 10 herausragende Ende des Überführblechs 21 schließt sich eine nicht dargestellte Transporteinrichtung der bereits erwähnten Füll- und Verschließmaschine an.

Erfindungswesentlich ist eine innerhalb des Kühlteils 13 des Sterilisiertunnels 10 angeordnete Leerfahreinrichtung 25. Die Leerfahreinrichtung 25 weist einen in einer horizontalen Ebene sich erstreckenden Rahmen 26 aus vorzugsweise strömungsgünstig ausgebildeten Profilstäben 27 auf, um der in der Kühlzone 13 herrschenden laminaren Luftstrom 18 wenig Widerstand entgegenzusetzen bzw. diese möglichst wenig zu stören. Die Profilstäbe 27 bilden im Ausführungsbeispiel einen Rechteckrahmen aus. Der Rahmen 26 besitzt vier Aufhängepunkte 28, 29, von denen sich die beiden vorderen Aufhängepunkte 28 im Bereich der Ecken auf der dem Ende 14 entgegengesetzten Seite des Rahmens 26 befinden. Die beiden anderen Aufhängepunkte 29 sind in etwa in der Mitte des Rahmens 26 angeordnet, wobei sich der Schwerpunkt des Rahmens 26 jedoch zwischen den Aufhängepunkten 28, 29 befindet. An den Aufhängepunkten 28, 29 sind Drähte 31, 32 oder aber Stahlbänder befestigt. Die den vorderen Aufhängepunkten 28 zugeordneten Drähte 31 sind mit einer Welle 33 verbunden, die sich unterhalb der Filterelemente 17 der Kühlzone 13 auf der der Sterilisierzone 12 zugewandten Seite befindet. Die Welle 33 ist mit einem nicht dargestellten Antrieb gekoppelt, der eine Drehung der Welle 33 in beide Richtungen ermöglicht. Mit der Welle 33 sind auch die beiden den anderen Aufhängepunkten 29 zugeordneten Drähte 32 verbunden. Diese Drähte 32 sind jedoch um eine parallel zur Welle 33 unterhalb der Filterelemente 17 angeordneten Achse 34 geführt. Die Länge der Drähte 31, 32 ist so aufeinander abgestimmt, daß bei einer Drehung der Welle 33 der Rahmen 26 parallel senk- bzw. hebbar ist.

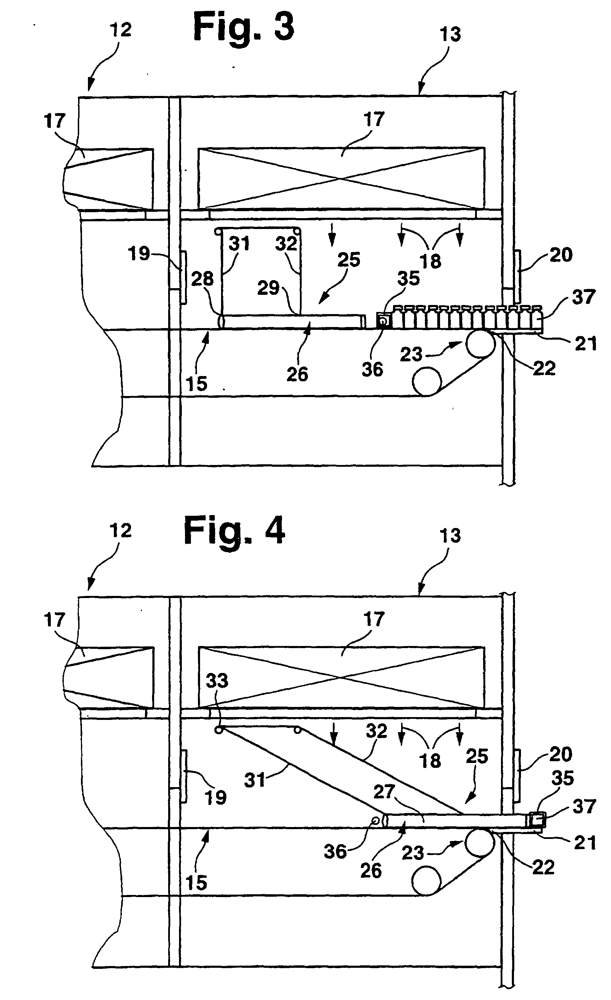

Die oben beschriebene Leerfahreinrichtung 25 arbeitet wie folgt: Nachdem die letzten Vials 1 einer Charge oder am Ende einer Produktionsschicht der Einlaufzone 11 des Sterilisiertunnels 10 zugeführt wurden, wird unmittelbar an die letzten Vials 1 ein das Transportband 15 quer überdeckender, balkenförmiger Leerfahrschieber 35 an die Vials 1 angeschlossen. Dieser Leerfahrschieber 35 wird von dem Transportband 15 zusammen mit den letzten Vials 1 durch den Sterilisiertunnel 10 gefördert (Figur 2). Vorzugsweise sobald sich der Leerfahrschieber 35 an einer bestimmten Stelle unterhalb des Rahmens 26 befindet, was mittels eines ersten, mit der Steuereinrichtung des Sterilisiertunnels 10 gekoppleten Sensors 36 erfasst wird, wird damit begonnen, den Rahmen 26 durch eine entsprechende Drehung der Welle 33 abzusenken. Im günstigsten Fall schließt sich der auf das Transportband 15 abgesenkte Rahmen 26 nahezu unmittelbar an der Leerfahrschieber 35 an (Figur 3).

Sobald der Rahmen 26 auf das Transportband 15 abgesenkt ist, wird die Welle 33 von ihrem Antrieb entkoppelt oder der Antrieb stromlos geschaltet, so daß der Rahmen 26 vom Transportband 15 mitgenommen werden kann, ohne daß sich der Abstand zum Leerfahrschieber 35 wieder vergrößert. Dadurch, daß der Rahmen 26 eine gewisse Masse besitzt bzw. daß zwischen dem Rahmen 26 und dem Transportband 15 ein hoher Haftreibungsfaktor herrscht, der zum Beispiel mittels einer Beschichtung der Unterseite des Rahmens 26 noch erhöht werden kann, schiebt das vorauseilende Ende des Rahmens 26 den Leerfahrschieber 35 samt der vor diesem befindlichen Vials 1 über den Umlenkbereich 23 des Transportbandes 15 auf das Überführblech 21 und durch die Austrittsschleuse 20 der Kühlzone 13 (Figur 4). Da die Aufhängepunkte 29 nicht in den freien Ecken des Rahmens 26, sondern in etwa in dessen Mitte angeordnet sind, ist es möglich, daß das vorauseilende Ende des Rahmens 26 durch die Austrittsschleuse 20 hindurchtritt, ohne daß dadurch wegen der Drähte 32 die Austrittschleuse 20 angehoben werden muß.

Sobald der Leerfahrschieber 35 vom Rahmen 26 durch die Austrittsschleuse 20 hindurchgeschoben ist, was mittels eines zweiten mit der Steuereinrichtung des Sterilisiertunnels 10 gekoppelten Sensors 37 erfassbar ist, sollte der Antrieb des Transportbandes 15 abgeschaltet oder sogar in umgekehrter Richtung betrieben werden, um das nachfolgende Rückführen des Rahmens 26 in seine ursprüngliche, angehobene Position zu erleichtern. Bei diesem Rückführvorgang wird die Welle 33 nun in entgegengesetzter Richtung gedreht. Dabei spulen sich die Drähte 31, 32 auf die Welle 33 auf und heben den Rahmen 26 vom Transportband 15 ab, sobald sich die Aufhängepunkte 28, 29 unterhalb der Welle 33 und der Achse 34 befinden.

## Patentansprüche

1. Sterilisiertunnel (10), mit einer pharmazeutische Behälter (1) durch eine Einlaufzone (11), eine Sterilisierzone (12) und eine Kühlzone (13) fördernden Transporteinrichtung, die als endloses Förderband (15) mit einem in der Kühlzone (13) angeordneten Umlenkbereich (23) ausgebildet ist, mit einem an den Umlenkbereich (23) anschließenden Überführelement (21), auf dem die pharmazeutischen Behälter (1) durch den Staudruck der noch auf dem Förderband (15) geförderten nachfolgenden Behälter (1) entlanggleiten, wobei das Überführelement (21) die Kühlzone (13) im Bereich einer Schleuse (20) durchdringt, mit einem das Förderband (15) quer überdeckenden Leerfahrschieber (35), der zum Leerfahren des Sterilisiertunnels (10) von dem Förderband (15) durch die Zonen (11, 12, 13) förderbar ist und mit Mitteln zum Weiterfördern des Leerfahrschiebers (35) durch die Schleuse (20), **dadurch gekennzeichnet, daß** oberhalb des Förderbandes (15) eine heb- und senkbare Einrichtung (25) zum Ausschleusen des Leerfahrschiebers (35) aus der Kühlzone (13) angeordnet ist, die zum Ausschleusen auf das Förderband (15) absenkbar ist, welches die Einrichtung (25) in Kontakt mit dem Leerfahrschieber (35) bringt und diesen anschließend auf das Überführelement (21) und durch die Schleuse (20) schiebt.

2. Sterilisiertunnel nach Anspruch 1, **dadurch gekennzeichnet, daß** die Einrichtung (25) unterhalb von Filterelementen (17) in einem senkrecht zur Förderrichtung der Behälter (1) ausgerichteten Luftstrom (18) angeordnet ist und einen aus Stäben (27) gebildeten Rahmen (26) aufweist.

3. Sterilisiertunnel nach Anspruch 1 oder 2, **dadurch gekennzeichnet, daß** die Einrichtung (25) Aufhängepunkte (28, 29) für Drahtelemente (31, 32) aufweist, die mittels wenigstens einer mit einem Antrieb gekoppelten Welle (33) auf- und abspulbar sind.

## Claims

1. Sterilization tunnel (10), having a transport device, which conveys pharmaceutical containers (1) through an inlet zone (11), a sterilization zone (12) and a cooling zone (13) and is designed as an endless conveyor belt (15) having a diverter region (23) arranged in the cooling zone (13), having a transfer element (21), which adjoins the diverter region (23) and along which the pharmaceutical containers (1) slide as a result of the dynamic pressure exerted by the subsequent containers (1) which are still being conveyed on the conveyor belt (15), the transfer element (21) penetrating through the cooling zone (13) in the region of a lock (20), having an empty-running slide (35), which transversely covers the conveyor belt (15) and can be conveyed through the zones (11, 12, 13) by the conveyor belt (15) in order to run the sterilization tunnel (10) empty, and having means for conveying the empty-running slide (35) onwards through the lock (20), **characterized in that** a raiseable and lowerable device (25) for discharging the empty-running slide (35) from the cooling zone (13) through a lock is arranged above the conveyor belt (15) and can be lowered onto the conveyor belt (15) in order for the empty-running slide to be discharged through a lock, so that the conveyor belt brings the device (25) into contact with the empty-running slide (35) and then pushes the latter onto the transfer element (21) and through the lock (20).

2. Sterilization tunnel according to Claim 1, **characterized in that** the device (25) is arranged below filter elements (17) in an air stream (18) directed perpendicular to the conveying direction of the containers (1) and has a frame (26) formed from rods (27).

3. Sterilization tunnel according to Claim 1 or 2, **characterized in that** the device (25) has suspension points (28, 29) for wire elements (31, 32), which can be wound up and unwound by means of at least one shaft (33) coupled to a drive.

## Revendications

1. Tunnel de stérilisation (10) comprenant un dispositif de transport qui transporte des récipients pharmaceutiques (1) à travers une zone d'entrée (11), une zone de stérilisation (12) et une zone de refroidissement (13), et qui est constitué par une bande transporteuse sans fin (15) comprenant une région de transfert (23) disposée dans la zone de refroidissement (13), un élément de transfert (21) adjacent à la région de transfert (23), sur lequel les récipients pharmaceutiques (1) glissent sous la pression dynamique des récipients (1) suivants qui sont encore transportés sur la bande transporteuse (15), l'élément de transfert (21) traversant la zone de refroidissement (13) dans la région d'un sas (20), un poussoir d'évacuation (35) recouvrant transversalement la bande transporteuse (15) qui peut être entraîné par la bande transporteuse (15) à travers les zones (11, 12, 13) pour l'évacuation du tunnel de stérilisation (10), et des moyens pour continuer à faire avancer le poussoir d'évacuation (35) à travers le sas (20),
**caractérisé en ce qu'**
au-dessus de la bande transporteuse (15), est disposé un dispositif (25) pouvant être levé et abaissé, servant à expulser le poussoir d'évacuation (35) de la zone de refroidissement (13), et qui peut être abaissé sur la bande transporteuse (15) pour l'expulsion, laquelle bande met le dispositif (25) en contact avec le poussoir d'évacuation (35) et, ensuite, pousse ce dernier sur l'élément de transfert (21) et à travers le sas (20).

2. Tunnel de stérilisation selon la revendication 1,
**caractérisé en ce que**
le dispositif (25) est disposé au-dessous d'éléments filtrants (17) dans un flux d'air (18) orienté perpendiculairement à la direction du transport des récipients (1), et présente un châssis (26) formé de barres (27).

3. Tunnel de stérilisation selon la revendication 1 ou 2,
**caractérisé en ce que**
le dispositif (25) présente des points d'accrochage (28, 29) pour des éléments en fil (31, 32) qui peuvent être enroulés et déroulés au moyen d'au moins un arbre (33) accouplé à un entraînement.
